# EUROPEAN PATENT APPLICATION

(11) **EP 3 988 030 A1**
(43) Date of publication of application: **27.04.2022**
(21) Application number: 20831879.0
(22) Date of filing: 10.06.2020
(51) Int. Cl.: A61B 8/12, A61B 17/00, A61M 25/00

(54) **CATHETER, CATHETER SET, MEDICAL DEVICE, AND ULTRASONIC MEASUREMENT METHOD**

(30) Priority: 24.06.2019 JP 2019116508
(71) Applicant: ASAHI INTECC CO., LTD., Seto-shi, Aichi 489-0071 (JP)
(72) Inventor: NAMIMA Satoshi, Seto-shi, Aichi 489-0071 (JP); OSHIMA Fumiyoshi, Seto-shi, Aichi 489-0071 (JP); KUBO Yuta, Seto-shi, Aichi 489-0071 (JP)
(74) Representative: Winter, Brandl - Partnerschaft mbB
(86) International application number: PCT/JP2020/022935
(87) International publication number: WO 2020/262001

(57) **Abstract**

To provide a catheter, a catheter set, a medical instrument, and an ultrasonic measurement method capable of accurately measuring a thickness of an object, without excessively pressing the object.

A catheter 1 according to the present disclosure is used with any one of a distal end 11t of an inner shaft 11 and a distal end 21t of an outer shaft 21 contacting an object b and includes the inner shaft 11 including a lumen 11h along a longitudinal axis direction, the outer shaft 21 provided to cover an outer periphery of the inner shaft 11, and an ultrasonic element 31 that is located between the inner shaft 11 and the outer shaft 21, is arranged at a proximal end side from at least any one of the distal end 11t of the inner shaft 11 and the distal end 21t of the outer shaft 21, and is capable of transmitting and receiving ultrasonic waves.

## Description

### TECHNICAL FIELD

The present invention relates to a catheter, a catheter set, a medical instrument, and an ultrasonic measurement method.

### BACKGROUND ART

A known example of an instrument for measuring the thickness of tissues and lesions of blood vessels or the like includes a catheter provided with an ultrasonic transceiver.

In such a catheter, a reflected wave of an ultrasonic wave emitted from an ultrasonic generator toward tissue or the like is received by an ultrasonic transducer, and the thickness of the tissue or the like is measured based on a time required for the reflection of the ultrasonic wave. In a catheter using such a technique, a contact force sensor is embedded in a distal end portion of the catheter, for example (see Patent Literature 1, for example).

According to the above-described catheter, if a contact force on an object obtained by the contact force sensor and a reflected wave from the object obtained by the ultrasonic transducer are measured, it is possible to measure the thickness of the object, based on a correlation between the contact force and the reflected wave.

### CITATION LIST

### Patent Literature

[Patent Literature 1] JP2016-123868A

### SUMMARY OF INVENTION

### TECHNICAL PROBLEM

However, in the conventional catheter as described above, the correlation between the contact force and the reflected wave is used for measurement, and thus, it is necessary to reciprocate the catheter while pressing the catheter against the object. Therefore, the movement of the catheter may be complicated during measurement, or the object may be deformed by some pressing methods, which may hinder smooth and accurate measurement.

The present invention has been contrived on the basis of the circumstances described above, and an object thereof is to provide a catheter, a catheter set, a medical instrument, and an ultrasonic measurement method capable of accurately measuring a thickness of an object, without excessively pressing the object.

### SOLUTION TO PROBLEM

Some aspects of the present disclosure are
(1) a catheter used with any one of a distal end of an inner shaft and a distal end of an outer shaft contacting an object, the catheter comprising
   an inner shaft including a lumen along a longitudinal axis direction,
   an outer shaft provided to cover an outer periphery of the inner shaft, and
   an ultrasonic element that is located between the inner shaft and the outer shaft, is arranged on a proximal end side from at least any one of the distal end of the inner shaft and the distal end of the outer shaft, and is capable of transmitting and receiving ultrasonic waves,
(2) the catheter according to (1), in which the distal end of the outer shaft is located on a distal end side from the distal end of the inner shaft,
(3) the catheter according to (1) or (2) above, in which the ultrasonic element includes a plurality of ultrasonic elements, and
   the plurality of ultrasonic elements are arranged along a circumferential direction of the outer periphery of the inner shaft,
(4) a catheter set including
   the catheter according to any one of (1) to (3) above, and
   a therapeutic device being inserted into the lumen of the inner shaft and including a distal end portion capable of advancing further to a distal end side than the distal end of the inner shaft,
(5) an ultrasonic measurement method used with any one of a distal end of an inner shaft and a distal end of an outer shaft contacting an object, the ultrasonic measurement method using a catheter, the catheter including
   an inner shaft including a lumen along a longitudinal axis direction,
   an outer shaft provided to cover an outer periphery of the inner shaft, and
   an ultrasonic element that is located between the inner shaft and the outer shaft, is arranged on a proximal end side from at least any one of the distal end of the inner shaft and the distal end of the outer shaft, and is capable of transmitting and receiving ultrasonic waves, the ultrasonic measurement method including
   a first step of transmitting an ultrasonic wave from the ultrasonic element,
   after the first step, a second step of receiving, by the ultrasonic element, a first reflected wave reflected off a surface of the object and measuring a first time from a transmission of the ultrasonic wave to a reception of the first reflected wave,
   after the second step, a third step of determining whether the distal end of the inner shaft and/or the distal end of the outer shaft contacts the surface of the object, based on the first time and a longer distance between a distance from an arrangement position of the ultrasonic element to the distal end of the inner shaft and a distance from the arrangement position of the ultrasonic element to the distal end of the outer shaft,
(6) the ultrasonic measurement method according to (5) above, including
   after confirming that the distal end of the inner shaft and/or the distal end of the outer shaft contacts the surface of the object in the third step, a fourth step of receiving, by the ultrasonic element, a second reflected wave reflected by a deep portion deeper than the surface of the object, and measuring a second time from the transmission of the ultrasonic wave to a reception of the second reflected wave, and
   after the fourth step, a fifth step of calculating a thickness of the object, based on the first time and the second time,
(7) the ultrasonic measurement method according to (5) or (6) above, including
   after confirming that the distal end of the inner shaft and/or the distal end of the outer shaft contacts the surface of the object in the third step, a sixth step of inserting an insertion body into the object via the lumen of the inner shaft, and
   after the sixth step, a seventh step of receiving, by the ultrasonic element, a third reflected wave reflected by the insertion body and measuring a third time from the transmission of the ultrasonic wave to a reception of the third reflected wave,
(8) the ultrasonic measurement method according to (7) above, including
   after the seventh step, an eighth step of determining whether an insertion depth of the insertion body into the object is appropriate, based on the first time, the second time, and the third time, and
(9) a medical instrument including
   the catheter according to any one of (1) to (3) above,
   a therapeutic device being inserted into a lumen of the inner shaft and including a distal end portion capable of advancing further to a distal end side than the distal end of the inner shaft,
   a first drive device that advances and retreats the catheter into and from a body cavity,
   a second drive device that advances and retreats the therapeutic device, and
   a control device that controls the first drive device and the second drive device.

It is noted that as used herein, the "distal end side" refers to a direction along the longitudinal axis direction of the inner shaft and a direction in which the object is located with respect to the inner shaft. Further, the "proximal end side" refers to a direction along the long axis direction of the inner shaft and a direction opposite to the distal end side. Further, the "distal end" refers to an end at the distal end side in any member or site, and the "proximal end" refers to an end at the proximal end side in any member or site, respectively.

### ADVANTAGEOUS EFFECTS OF INVENTION

The present invention can provide a catheter, a catheter set, a medical instrument, and an ultrasonic measurement method capable of accurately measuring a thickness of an object, without excessively pressing the object.

### BRIEF DESCRIPTION OF DRAWINGS

FIG. 1 is a schematic cross-sectional view illustrating a first embodiment of the present invention.
FIG. 2 is a schematic front view of FIG. 1.
FIG. 3A is a partially enlarged schematic cross-sectional view illustrating a modification of FIG. 1.
FIG. 3B is a partially enlarged schematic cross-sectional view illustrating a modification of FIG. 1.
FIG. 4A is a schematic front view illustrating a modification of FIG. 1.
FIG. 4B is a schematic front view illustrating a modification of FIG. 1.
FIG. 5 is a schematic cross-sectional view illustrating a second embodiment of the present invention.
FIG. 6 is a schematic view illustrating a third embodiment of the present invention.
FIG. 7A is an explanatory diagram of a fourth embodiment of the present invention.
FIG. 7B is an explanatory diagram of the fourth embodiment of the present invention.
FIG. 7C is an explanatory diagram of the fourth embodiment of the present invention.
FIG. 7D is an explanatory diagram of the fourth embodiment of the present invention.
FIG. 7E is an explanatory diagram of the fourth embodiment of the present invention.
FIG. 7F is an explanatory diagram of the fourth embodiment of the present invention.
FIG. 8A is a partially enlarged schematic cross-sectional view illustrating a modification of FIG. 1.
FIG. 8B is a partially enlarged schematic cross-sectional view illustrating a modification of FIG. 1.

### DESCRIPTION OF EMBODIMENTS

Embodiments of a catheter, a catheter set, a medical instrument, and an ultrasonic measurement method according to the present disclosure will be described below with reference to the drawings, but the present invention is not limited only to the embodiments described in the drawings. Further, the dimensions of the catheter, the catheter set, and the medical instrument illustrated in the drawings are dimensions indicated to facilitate the understanding of the contents of implementation and do not correspond to the actual dimensions.

### <Catheter>

The catheter is a catheter used with any one of a distal end of an inner shaft and a distal end of an outer shaft contacting an object and is characterized in including the inner shaft including a lumen along a longitudinal axis direction, the outer shaft provided to cover an outer periphery of the inner shaft, and an ultrasonic element that is located between the inner shaft and the outer shaft, is arranged on a proximal end side from at least any one of the distal end of the inner shaft and the distal end of the outer shaft, and is capable of transmitting and receiving ultrasonic waves.

### [First Embodiment]

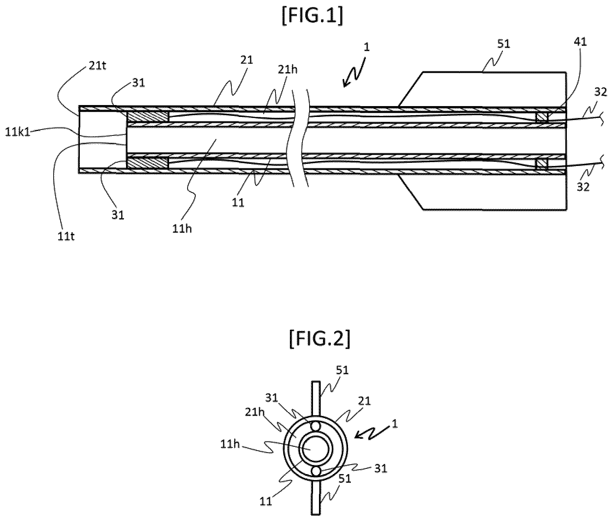

FIG. 1 is a schematic cross-sectional view illustrating a first embodiment of the present invention. FIG. 2 is a schematic front view of FIG. 1. As illustrated in FIGS. 1 and 2, a catheter 1 schematically includes an inner shaft 11, an outer shaft 21, an ultrasonic element 31, and a connector 51. It is noted that a method of using the catheter 1 will be described in detail in <Ultrasonic Measurement Method> described later.

The inner shaft 11 is a shaft including a lumen 11h along the longitudinal axis direction. Specifically, the inner shaft 11 may be formed of a hollow-shaped shaft penetrating from the distal end to the proximal end, for example. For example, a therapeutic device 71 (described later), a medical solution (not illustrated), or the like is inserted through the lumen 11h of the inner shaft 11.

The outer shaft 21 is a shaft provided to cover an outer periphery of the inner shaft 11. Specifically, the outer shaft 21 may be configured to hold the inner shaft 11 in a lumen 21h and cover the inner shaft 11 over the entire length of the inner shaft 11 along a longitudinal axis direction.

The outer shaft 21 is connected to the inner shaft 11. Examples of a method of connecting the outer shaft 21 and the inner shaft 11 may include a method in which a spacer member 41 is interposed between the outer shaft 21 and the inner shaft 11 and the outer shaft 21 and the inner shaft 11 are connected by the spacer member 41, a method in which a part of an inner peripheral surface of the outer shaft 21 and a part of an outer peripheral surface of the inner shaft 11 are joined by welding or the like, a method in which the outer shaft 21 and the inner shaft 11 are connected via the ultrasonic element 31 described later, and a method obtained by combining these methods. In the present embodiment, the outer shaft 21 and the inner shaft 11 are adhered (fixed) by an adhesive via the ultrasonic element 31 and the spacer member 41.

Materials forming the inner shaft 11 and the outer shaft 21 are not particularly limited as long as an effect of the present invention is not impaired. If the catheter 1 is used as a medical treatment device to be inserted into a body, materials forming each of the inner shaft 11 and the outer shaft 21 are preferably formed of materials having antithrombogenicity, flexibility, and biocompatibility, and an example of such materials include resin materials such as a polyamide resin, a polyolefin resin, a polyester resin, a polyurethane resin, a silicone resin, and a fluororesin.

Here, as for the dimensions of the inner shaft 11 and the outer shaft 21, for example, an inner diameter of the inner shaft 11 may be set to 0.5 mm to 1.5 mm, a length of the inner shaft 11 may be set to 800 mm to 1500 mm, an inner diameter of the outer shaft 21 may be set to 0.8 mm to 4 mm, and a length of the outer shaft 21 may be set to 800 mm to 1500 mm. It is noted that a space into which a later-described cord 32 for communication and power transmission extending from the ultrasonic element 31 can be inserted is preferably provided between the outer peripheral surface of the inner shaft 11 and the inner peripheral surface of the outer shaft 21.

The ultrasonic element 31 is an element that is located between the inner shaft 11 and the outer shaft 21, is arranged on a proximal end side from at least any one of a distal end 11t of the inner shaft 11 and a distal end 21t of the outer shaft 21, and is capable of transmitting and receiving ultrasonic waves. For example, as in the catheter 1, the ultrasonic element 31 may be fixed on an outer peripheral surface of a distal end portion of the inner shaft 11. An example of a method of connecting the ultrasonic element 31 to the inner shaft 11 and the outer shaft 21 may include a method in which the ultrasonic element 31 is adhesively fixed to each of the outer peripheral surface of the inner shaft 11 and the inner peripheral surface of the outer shaft 21 by an adhesive.

Examples of an ultrasonic element to be employed for the ultrasonic element 31 include an ultrasonic element separately including a wave transmitter that transmits ultrasonic waves toward an object and a wave receiver that receives a reflected wave from an object, and an ultrasonic element including one ultrasonic transducer that alternately switches between a wave transmission mode and a wave reception mode to transmit and receive waves. An example of a frequency of ultrasonic waves transmitted from the ultrasonic element 31 may include from 20 kHz to 1000 kHz. A pulse wave, a continuous wave, and the like may be employed for a waveform of the ultrasonic wave. The above pulse wave may be any one of a single pulse wave or a periodic or aperiodic pulse wave.

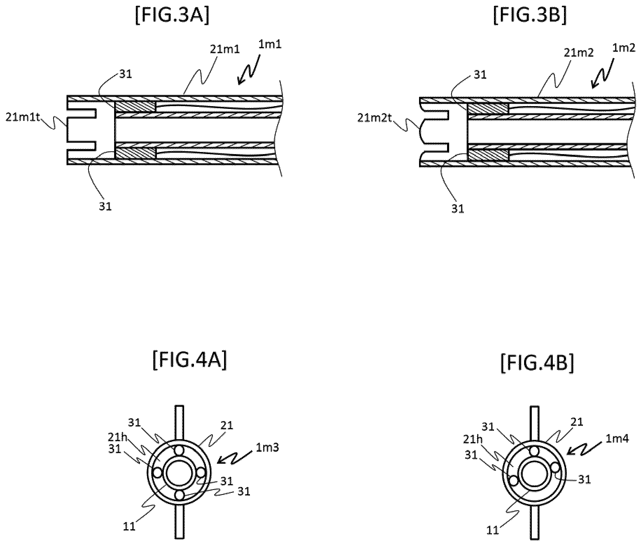

Here, as in the catheter 1, the distal end 21t of the outer shaft 21 is preferably located on a distal end side from the distal end 11t of the inner shaft 11 (the distal end 21t of the outer shaft 21 is formed to be separated toward the distal end side from the distal end 11t of the inner shaft 11). In such a case, the shape of the distal end 21t of the outer shaft 21 may include an annular flat surface orthogonal to the longitudinal axis direction as in the distal end 21t of the outer shaft 21 in the catheter 1 (see FIG. 1), an arc-shaped flat surface orthogonal to the longitudinal axis direction as in a distal end 21m1t of an outer shaft 21m1 in a catheter 1m1 (see FIG. 3A), and a convex curved surface curved toward the distal end side as in a distal end 21m2t of an outer shaft 21m2 in a catheter 1m2 (see FIG. 3B). It is noted that, in the catheter 1, the ultrasonic element 31 is fixed on the outer peripheral surface of the distal end portion of the inner shaft 11. The distal end 21t of the outer shaft 21 is located on the distal end side from the distal end 11t of the inner shaft 11 as described above. Therefore, a reflected ultrasonic wave can be captured on the distal end side from the distal end 11t of the inner shaft 11 without being blocked by the inner shaft 11 to sense a behavior in front of the inner shaft 11.

It is noted that the ultrasonic element 31 may include a plurality of ultrasonic elements (each ultrasonic element is also referred to as the ultrasonic element 31), and it is preferable that the plurality of ultrasonic elements 31 are arranged along a circumferential direction of an outer periphery of the inner shaft 11. Examples of such a catheter include the catheter 1 and a catheter 1m3 (see FIGs. 2 and 4A), in which two or more of the ultrasonic elements 31 are arranged at equal intervals in the circumferential direction along the outer periphery of the inner shaft 11, and a catheter 1m4 (see FIG. 4B) in which the ultrasonic elements 31 are arranged at different intervals. If the plurality of ultrasonic elements 31 are arranged along the circumferential direction of the outer periphery of the inner shaft 11, one reflected wave can be received by the plurality of ultrasonic elements 31, and thus, it is possible to better understand a positional relationship between an object and a distal end portion of the catheter 1 (for example, a tilt of the longitudinal axis of the inner shaft 11 with respect to a surface of the object), based on a difference in reception times.

The connector 51 is a member used by an operator to hold the catheter 1. The connector 51 may be connected to cover a proximal end portion of the outer shaft 21, for example. It is noted that the shape of the connector 51 is not particularly limited as long as an effect of the present invention is not impaired.

As described above, the catheter 1 has the above-described configuration. Accordingly, because the ultrasonic element 31 is arranged on the proximal end side from at least any one of the distal end 11t of the inner shaft 11 and the distal end 21t of the outer shaft 21, the object and the ultrasonic element 31 can be located away from each other. Therefore, the catheter 1 can surely determine a contact between the catheter 1 and the object without excessively pressing the object to accurately measure a thickness and the like of the object.

### <Catheter Set>

The catheter set includes the above-described catheter according to the present disclosure and a therapeutic device being inserted into the lumen of the inner shaft and having a distal end portion that can advance further to a distal end side than a distal end of the inner shaft.

### [Second Embodiment]

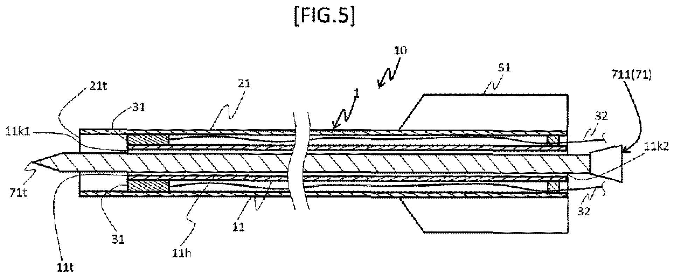

FIG. 5 is a schematic cross-sectional view illustrating a second embodiment of the present invention. As illustrated in FIG. 5, a catheter set 10 schematically includes the catheter 1 and the therapeutic device 71. It is noted that a configuration of the catheter 1 is similar to that in the first embodiment, and thus, the same parts as those of the first embodiment are designated by the same reference numerals, and detailed description thereof will be omitted here. Further, a method of using the catheter set 10 will be described in detail in <Ultrasonic Measurement Method> described later.

The catheter 1 is a catheter used with any one of the distal end 11t of the inner shaft 11 and the distal end 21t of the outer shaft 21 contacting an object and includes the inner shaft 11 provided with the lumen 11h along the longitudinal axis direction, the outer shaft 21 provided to cover an outer periphery of the inner shaft 11, and the ultrasonic element 31 that is located between the inner shaft 11 and the outer shaft 21, is arranged on a proximal end side from at least any one of the distal end 11t of the inner shaft 11 and the distal end 21t of the outer shaft 21, and is capable of transmitting and receiving ultrasonic waves.

The therapeutic device 71 is a therapeutic device that is inserted into the lumen 11h of the inner shaft 11 and includes a distal end portion that can advance further to a distal end side than the distal end 11t of the inner shaft 11. Specifically, for example, the therapeutic device 71 can be configured so that the therapeutic device 71 is inserted into the lumen 11h from the distal end 11t to the proximal end of the inner shaft 11, and a distal end 71t of the therapeutic device 71 advancing from an opening 11k1 of the distal end 11t of the inner shaft 11 protrudes toward the distal end side beyond the distal end 21t of the outer shaft 21. On the other hand, a proximal end of the therapeutic device 71 may be arranged to pass through the inner shaft 11 and be exposed from an opening 11k2 of the proximal end of the inner shaft 11.

Examples of the therapeutic device 71 include a puncture needle, a dilator, and a ablation device. In the present embodiment, a puncture needle 711 is described as an example of the therapeutic device 71.

Here, the ultrasonic element 31 described above can be used to recognize, for example, a positional relationship between the distal end portion of the therapeutic device 71 advancing from the opening 11k1 of the distal end 11t of the inner shaft 11 and/or the distal end 21t of the outer shaft 21, or a positional relationship between the distal end portion of the therapeutic device 71 and a surface of an object and/or a tissue inside the object and the like. Specifically, because an ultrasonic wave reflected by the therapeutic device 71 (for example, the distal end 71t of the therapeutic device 71) is detected, it is possible to recognize a position of the therapeutic device 71 (for example, a position of the distal end 71t with respect to the ultrasonic element 31 in the longitudinal axis direction), based on the time required for the ultrasonic wave to be reflected.

As described above, the catheter set 10 has the above-described configuration. Accordingly, it is possible to recognize positions of the distal end 11t of the inner shaft 11, the distal end 21t of the outer shaft 21, and the distal end portion (the distal end 71t or the like) of the therapeutic device 71 by ultrasonic measurement, and thus, a positional relationship between the distal end portion of the therapeutic device 71 and an object or the like can be accurately recognized. As a result, the therapeutic device 71 can be used to surely treat the object.

### <Medical Instrument>

A medical instrument includes the above-described catheter according to the present disclosure, a therapeutic device being inserted into a lumen of an inner shaft and having a distal end portion that can advance further to a distal end side than a distal end of the inner shaft, a first drive device that advances and retreats the catheter into and from a body cavity, a second drive device that advances and retreats the therapeutic device, and a control device that controls the first drive device and the second drive device.

### [Third Embodiment]

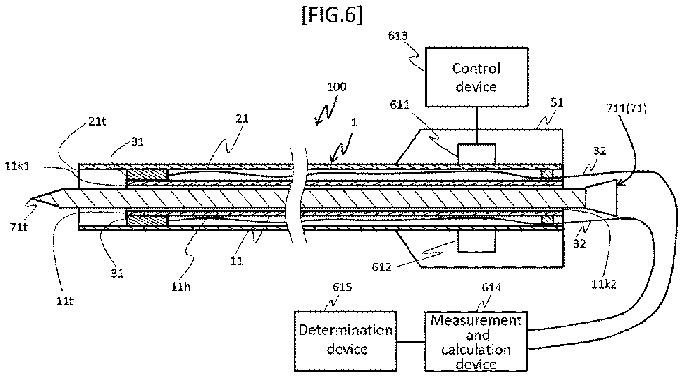

FIG. 6 is a schematic view illustrating a third embodiment of the present invention. As illustrated in FIG. 6, a medical instrument 100 schematically includes the catheter 1, the therapeutic device 71, a first drive device 611, a second drive device 612, a control device 613, a measurement and calculation device 614, and a determination device 615. It is noted that configurations of the catheter 1 and the therapeutic device 71 are similar to those in the first and second embodiments, and thus, the same parts as those of the first and second embodiments are designated by the same reference numerals, and detailed description thereof will be omitted here. Further, a method of using the medical instrument 100 will be described in detail later in the section <Ultrasonic Measurement Method>.

The catheter 1 is a catheter used with any one of the distal end 11t of the inner shaft 11 and the distal end 21t of the outer shaft 21 contacting an object and includes the inner shaft 11 provided with the lumen 11h along the longitudinal axis direction, the outer shaft 21 provided to cover an outer periphery of the inner shaft 11, and the ultrasonic element 31 that is located between the inner shaft 11 and the outer shaft 21, is arranged on a proximal end side from at least any one of the distal end 11t of the inner shaft 11 and the distal end 21t of the outer shaft 21, and is capable of transmitting and receiving ultrasonic waves.

The therapeutic device 71 is a therapeutic device that is inserted into the lumen 11h of the inner shaft 11 and includes a distal end portion that can advance further to the distal end side than the distal end 11h of the inner shaft 11. In the present embodiment, the puncture needle 711 is described as an example of the therapeutic device 71.

Here, the ultrasonic element 31 described above can be used to recognize, for example, a positional relationship between the distal end portion of the therapeutic device 71 advancing from the opening 11k1 of the distal end 11t of the inner shaft 11 and/or the distal end 21t of the outer shaft 21, or a positional relationship between the distal end portion of the therapeutic device 71 and a surface of an object and/or a tissue inside the object and the like. Specifically, because an ultrasonic wave reflected by the therapeutic device 71 (for example, the distal end 71t of the therapeutic device 71) is detected, it is possible to recognize a position of the therapeutic device 71 (for example, a position of the distal end 71t with respect to the ultrasonic element 31 in the longitudinal axis direction), based on the time required for the ultrasonic wave to be reflected.

The first drive device 611 is a device that advances and retreats the catheter 1 into and from a body cavity. The first drive device 611 is provided in the connector 51, for example, and may be configured to advance and retreat the catheter 1 along the longitudinal axis direction of the inner shaft 11 while engaging with the outer periphery of the outer shaft 21. A range in which the catheter 1 is advanced and retreated depends on a distance in which the catheter 1 is moved inside a body, but can be set to a range between a position where the connector 51 and the distal end portion of the catheter 1 coincide and a position where the connector 51 and the proximal end portion of the catheter 1 coincide, for example.

The second drive device 612 is a device that advances and retreats the therapeutic device. The second drive device 612 is provided in the connector 51, for example, and may be configured to advance and retreat the therapeutic device 71 in the lumen 11h of the inner shaft 11 along the longitudinal axis direction of the inner shaft 11 while engaging with the outer periphery of the therapeutic device 71. A range in which the therapeutic device 71 is advanced and retreated depends on a treatment content, but can be set to a range between a position where the distal end 71t of the therapeutic device 71 is within the inner shaft 11 and a position where the distal end 71t of the therapeutic device 71 exceeds the distal end 21t of the outer shaft 21 to enter the object (for example, a position of a treatment site).

For example, a linear actuator, a linear motor, and an ultrasonic motor can be employed for the first drive device 611 and the second drive device 612 described above.

The control device 613 is a device that controls the first drive device 611 and the second drive device 612. Specifically, the control device 613 controls the first drive device 611, for example, based on a distance from the ultrasonic element 31 to a furthermost end among the distal ends of the inner shaft 11 and the outer shaft 21 (distance-to-furthermost end) and a time until the ultrasonic element 31 receives a reflected wave reflected off a surface of an object, so that the distal end 11t of the inner shaft 11 and/or the distal end 21t of the outer shaft 21 contacts the surface of the object. Further, the control device 613 controls the second drive device 612, for example, based on a reflected wave reflected by a distal end of the therapeutic device 71 advancing from the opening 11k1 via the lumen 11h of the inner shaft 11, so that the distal end 71t of the therapeutic device 71 is located at a desired site.

The measurement and calculation device 614 measures, for example, a timing for transmitting and receiving ultrasonic waves by the ultrasonic element 31, measures first to third times described later using the measurement/calculation value 614, and to calculate a distance to each site, a thickness of the object, and the like using these measured times.

The determination device 615 uses a result calculated by the measurement and calculation device 614 to determine, for example, whether the distal end 11t of the inner shaft 11 and/or the distal end 21t of the outer shaft 21 contacts the surface of the object and whether an insertion depth of an insertion body into the object is appropriate.

As described above, if the medical instrument 100 has the above-described configuration. Accordingly, it is possible to recognize positions of the distal end 11t of the inner shaft 11, the distal end 21t of the outer shaft 21, and the distal end portion (the distal end 71t or the like) of the therapeutic device 71 by ultrasonic measurement, and thus, a positional relationship between the distal end portion of the therapeutic device 71 and an object or the like can be accurately recognized. As a result, the therapeutic device 71 can be used to surely treat the object.

### <Ultrasonic Measurement Method>

The ultrasonic measurement method is an ultrasonic measurement method used with any one of a distal end of an inner shaft and a distal end of an outer shaft contacting an object. The ultrasonic measurement method includes, using the catheter according to the present disclosure,
(S1) a first step of transmitting an ultrasonic wave from an ultrasonic element,
(S2) after the first step, a second step of receiving, by the ultrasonic element, a reflected wave (a first reflected wave) reflected off a surface of the object and measuring a time (first time) from the transmission of the ultrasonic wave to the reception of the first reflected wave, and
(S3) after the second step, a third step of determining whether the distal end of the inner shaft and/or the distal end of the outer shaft contacts the surface of the object, based on the first time and a longer distance between a distance from an arrangement position of the ultrasonic element to the distal end of the inner shaft and a distance from the arrangement position of the ultrasonic element to the distal end of the outer shaft.

Further, the ultrasonic measurement method preferably includes (S4) after confirming that the distal end of the inner shaft and/or the distal end of the outer shaft contacts the surface of the object in the third step, a fourth step of receiving, by the ultrasonic element, a reflected wave (a second reflected wave) reflected by a deep portion deeper than the surface of the object, and measuring a time (second time) from the transmission of the ultrasonic wave to the reception of the second reflected wave, and
(S5) after the fourth step, a fifth step of calculating a thickness of the object, based on the first time and the second time.

Further, the ultrasonic measurement method preferably includes (S6) after confirming that the distal end of the inner shaft and/or the distal end of the outer shaft contacts the surface of the object in the third step, a sixth step of inserting an insertion body into the object via a lumen of the inner shaft, and
(S7) after the sixth step, a seventh step of receiving, by the ultrasonic element, a reflected wave (a third reflected wave) reflected by the insertion body and measuring a time (third time) from the transmission of the ultrasonic wave to the reception of the third reflected wave.

Further, the ultrasonic measurement method preferably includes (S8) after the seventh step, an eighth step of determining whether an insertion depth of the insertion body into the object is appropriate, based on the first time, the second time, and the third time.

The ultrasonic measurement method can be suitably applied to uses of the catheter 1, the catheter set 10, and the medical instrument 100 described above. Therefore, the catheter 1, the catheter set 10, and the medical instrument 100 are described as examples, but the application of the ultrasonic measurement method is not limited only to the use of the catheter 1, the catheter set 10, and the medical instrument 100.

### [Fourth Embodiment]

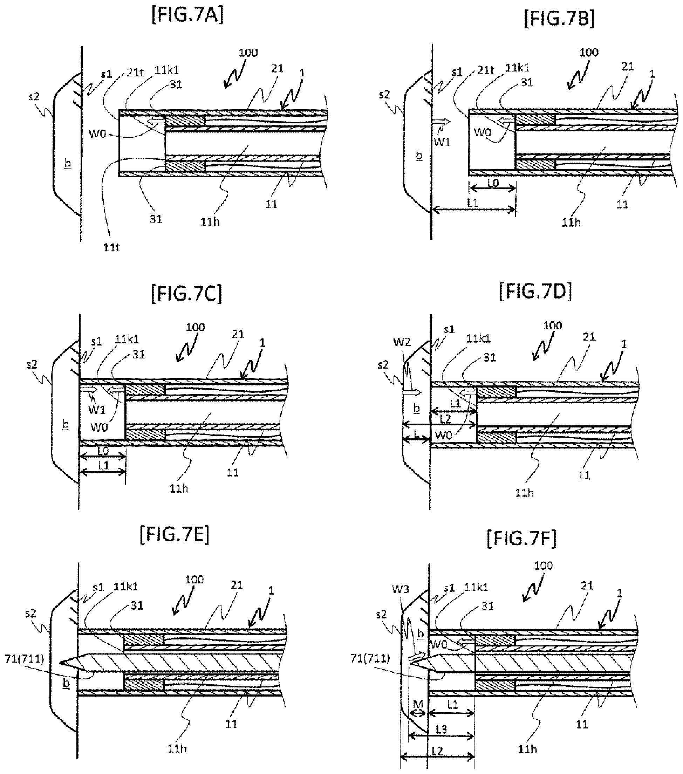

An embodiment of the ultrasonic measurement method will be described below with reference to FIGs. 7A to 7F. It is noted that the therapeutic device 71 (the puncture needle 711) is described herein as an example of the insertion body.

### (First Step S1)

In step S1, a catheter is used to transmit ultrasonic waves from an ultrasonic element. In the first step S1, specifically, for example, after inserting the catheter 1 into a blood vessel from a distal end of the catheter 1, an operator operates the control device 613 while grasping the connector 51, to operate the first drive device 611 and push ahead the distal end of the catheter 1 to the vicinity of an object b. Subsequently, for example, a periodic pulse wave (an ultrasonic wave W0) having a predetermined frequency is transmitted from the ultrasonic element 31 substantially toward the distal end side (see FIG. 7A).

### (Second Step S2)

In step S2, after the first step S1, a first reflected wave reflected by a surface of an object is received by the ultrasonic element and a first time from the transmission of the ultrasonic wave to the reception of the first reflected wave is measured. Specifically, in the second step S2, for example, the ultrasonic element 31 is used to receive a first reflected wave W1 of the ultrasonic wave W0 transmitted in the first step S1 (see FIG. 7B) and the measurement and calculation device 614 is used to measure a first time T1 from the transmission of the ultrasonic wave to the reception of the first reflected wave W1 by the ultrasonic element 31.

### (Third Step S3)

In step S3, after the second step S2, it is determined whether the distal end of the inner shaft and/or the distal end of the outer shaft contacts the surface of the object, based on the first time and a longer distance between a distance from an arrangement position of the ultrasonic element to the distal end of the inner shaft and a distance from the arrangement position of the ultrasonic element to the distal end of the outer shaft. In the third step S3, specifically, for example, the determination device 615 is used to compare a distance from the ultrasonic element 31 to a furthermost end among the distal ends of the inner shaft 11 and the outer shaft 21 (a distance-to-furthermost end L0), with a distance between the ultrasonic element 31 and a surface s1 of the object b (a first distance L1) obtained from the first time T1. At this time, if the first distance L1 is larger than the distance-to-furthermost end L0 (L1 > L0), the determination device 615 determines that the distal end 11t of the inner shaft 11 and the distal end 21t of the outer shaft 21 are separated from the object b (see FIG. 7B). On the other hand, if the first distance L1 and the distance-to-furthermost end L0 are the same (L1 = L0), the determination device 615 determines that the distal end 11t of the inner shaft 11 and/or the distal end 21t of the outer shaft 21 contacts the object (see FIG. 7C).

As described above, the ultrasonic measurement method includes the first step S1, the second step S2, and the third step S3, and thus, it is possible to surely determine whether the object b contacts the distal end 11t of the inner shaft 11 and/or the distal end 21t of the outer shaft 21 without excessively pressing the object b.

### (Fourth Step S4)

In step S4, after confirming that the distal end of the inner shaft and/or the distal end of the outer shaft contacts the surface of the object in the third step S3, a second reflected wave reflected by a deep portion deeper than the surface of the object, is received by the ultrasonic element and a second time from the transmission of the ultrasonic wave to the reception of the second reflected wave is measured. Specifically, in the fourth step S4, for example, the ultrasonic element 31 is used to receive a second reflected wave W2 formed when the ultrasonic wave W0 transmitted in the first step S1 is reflected off an interface s2 of a deep portion (an interface on a distal end side from the surface s1 of the object b) (see FIG. 7D), and the measurement and calculation device 614 is used to measure a second time T2 from the transmission of the ultrasonic wave W0 to the reception of the second reflected wave W2 by the ultrasonic element 31.

### (Fifth Step S5)

In step S5, after the fourth step S4, a thickness of the object is calculated based on the first time and the second time. In the fifth step S5, specifically, for example, the measurement and calculation device 614 uses the first time T1 measured in the second step S2 and the second time T2 measured in the fourth step S4 to calculate a difference (L2 - L1) between the first distance L1 converted from the first time T1 and a distance (a second distance L2) converted from the second time T2, and then, calculates a thickness L of the object (see FIG. 7D). It is noted that the second reflected wave received in the fourth step is a reflected wave reflected from an opposite surface in a depth direction facing the surface of the object against which the distal end of the inner shaft and/or the distal end of the outer shaft abuts, or from a lesion existing inside a wall surface of the object. Therefore, a depth of the lesion can be calculated using the second reflected wave, for example.

As described above, the ultrasonic measurement method further includes the fourth step S4 and the fifth step S5 described above, and thus, the thickness L of the object b can be accurately measured without excessively pressing the object b, and for example, a shape of the lesion and/or a depth of the lesion can be detected more accurately.

### (Sixth Step S6)

In step S6, after confirming that the distal end of the inner shaft and/or the distal end of the outer shaft contacts the surface of the object in the third step S3, the insertion body is inserted into the object via the lumen of the inner shaft. In the sixth step S6, specifically, for example, after inserting the therapeutic device 71 (the puncture needle 711) from the opening 11k2 at the proximal end of the inner shaft 11 (see FIG. 6), the operator operates the control device 613 while grasping the connector 51, to operate the second drive device 612 and advance the distal end portion of the therapeutic device 71 from the opening 11k1 at the distal end 11t of the inner shaft 11. Next, the second drive device 612 advances the distal end 11t of the inner shaft 11 to puncture the object b (see FIG. 7E).

### (Seventh Step S7)

In step S7, after the sixth step S6, a third reflected wave reflected by the insertion body is received by the ultrasonic element and a third time from the transmission of the ultrasonic wave to the reception of the third reflected wave is measured. Specifically, in the seventh step S7, for example, a third reflected wave W3 of an ultrasonic wave transmitted from the ultrasonic element 31 and reflected by the distal end 71t of the therapeutic device 71 is received (see FIG. 7F) and the measurement and calculation device 614 is used to measure a third time T3 from the transmission of the ultrasonic wave W0 to the reception of the third reflected wave W3 by the ultrasonic element 31. Further, in the seventh step S7, a difference (L3 - L1) between the first distance L1 converted from the first time T1 measured in the second step S2 and a distance (a third distance L3) converted from the above-mentioned third time T3 is calculated to confirm that the distal end 71t punctures the surface s1 of the object b.

As described above, the ultrasonic measurement method further includes the sixth step S6 and the seventh step S7 described above, and thus, it is possible to surely recognize whether the distal end 71t of the therapeutic device 71 enters the surface s1 of the object b, based on the first and third times T1 and T3.

### (Eighth Step S8)

In step S8, after the seventh step S7, it is determined whether an insertion depth of the insertion body into the object is appropriate, based on the first time, the second time, and the third time. In the eighth step S8, specifically, for example, a difference (L2 - L1) between the first distance L1 converted from the first time T1 measured in the second step S2 and the second distance L2 converted from the second time T2 measured in the fourth step S4, and the difference (L3 - L1) between the first distance L1 converted from the first time T1 measured in the second step S2 and the third distance L3 converted from the third time T3 measured in the seventh step S7, are used to evaluate a position (a depth M) of the distal end 71t of the therapeutic device in the object b. Next, the determination device 615 is used to determine whether the evaluated position of the distal end 71t is within a predetermined range (see FIG. 7F).

As described above, the ultrasonic measurement method further includes the eighth step S8, and thus, the position of the therapeutic device 71 (the puncture needle 711) (in particular, the position of the distal end 71t of the therapeutic device ) in the object b can be surely recognized, based on the first to third times T1 to T3. As a result, the therapeutic device 71 can be used to more accurately treat the lesion or the like (the object b).

It is noted that after treatment using the therapeutic device 71 at a desired position in the object b based on the determination in the eighth step S8, the second drive device 612 is used to remove the therapeutic device 71 from the object b, and further, the first drive device 611 is used to remove the catheter 1 from the body, and thereby, a procedure using the catheter 1, the catheter set 10, and the medical instrument 100 is completed.

The present invention is not limited to the configuration of the above-described embodiments, but is indicated by the claims, and is intended to include all modifications within meanings and the scope equivalent to the claims.

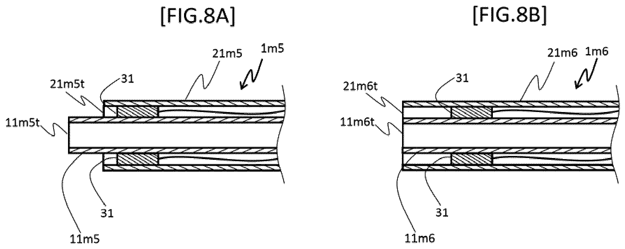

For example, in the first embodiment described above, the catheter 1 in which the distal end 21t of the outer shaft 21 is located on the distal end side from the distal end 11t of the inner shaft 11 is described. However, a catheter 1m5 in which a distal end 11m5t of an inner shaft 11m5 is located on the distal end side from a distal end 21m5t of an outer shaft 21m5 (see FIG. 8A) and a catheter 1m6 in which a position of a distal end 21m6t of an outer shaft 21m6 and a position of a distal end 11m6t of an inner shaft 11m6 are the same in the longitudinal axis direction (see FIG. 8B) can be employed.

Further, in the first embodiment described above, the catheter 1 including the plurality of ultrasonic elements 31 arranged along the circumferential direction of the outer periphery of the inner shaft 11 is described. However, a catheter including only one ultrasonic element (not illustrated) may also be employed.

### REFERENCE SIGNS LIST

- b: Object
- 1: Catheter
- 11: Inner shaft
- 11h: Lumen
- 21: Outer shaft
- 31: Ultrasonic element
- 611: First drive device
- 612: Second drive device
- 613: Control device
- 71: Therapeutic device (insertion body)
- 10: Catheter set
- 100: Medical instrument

## Claims

1. A catheter used with any one of a distal end of an inner shaft and a distal end of an outer shaft contacting an object, the catheter comprising:
an inner shaft including a lumen along a longitudinal axis direction;
an outer shaft provided to cover an outer periphery of the inner shaft; and
an ultrasonic element that is located between the inner shaft and the outer shaft, is arranged on a proximal end side from at least any one of the distal end of the inner shaft and the distal end of the outer shaft, and is capable of transmitting and receiving ultrasonic waves.

2. The catheter according to claim 1, wherein the distal end of the outer shaft is located on a distal end side from the distal end of the inner shaft.

3. The catheter according to claim 1 or 2, wherein the ultrasonic element includes a plurality of ultrasonic elements, and
the plurality of ultrasonic elements are arranged along a circumferential direction of the outer periphery of the inner shaft.

4. A catheter set, comprising:
the catheter according to any one of claims 1 to 3; and
a therapeutic device being inserted into the lumen of the inner shaft and including a distal end portion capable of advancing further to a distal end side than the distal end of the inner shaft.

5. An ultrasonic measurement method used with any one of a distal end of an inner shaft and a distal end of an outer shaft contacting an object, the ultrasonic measurement method using a catheter, the catheter including:
an inner shaft including a lumen along a longitudinal axis direction;
an outer shaft provided to cover an outer periphery of the inner shaft; and
an ultrasonic element that is located between the inner shaft and the outer shaft, is arranged on a proximal end side from at least any one of the distal end of the inner shaft and the distal end of the outer shaft, and is capable of transmitting and receiving ultrasonic waves, the ultrasonic measurement method comprising:
a first step of transmitting an ultrasonic wave from the ultrasonic element;
after the first step, a second step of receiving, by the ultrasonic element, a first reflected wave reflected off a surface of the object and measuring a first time from a transmission of the ultrasonic wave to a reception of the first reflected wave;
after the second step, a third step of determining whether the distal end of the inner shaft and/or the distal end of the outer shaft contacts the surface of the object, based on the first time and a longer distance between a distance from an arrangement position of the ultrasonic element to the distal end of the inner shaft and a distance from the arrangement position of the ultrasonic element to the distal end of the outer shaft.

6. The ultrasonic measurement method according to claim 5, comprising:
after confirming that the distal end of the inner shaft and/or the distal end of the outer shaft contacts the surface of the object in the third step, a fourth step of receiving, by the ultrasonic element, a second reflected wave reflected by a deep portion deeper than the surface of the object, and measuring a second time from the transmission of the ultrasonic wave to a reception of the second reflected wave; and
after the fourth step, a fifth step of calculating a thickness of the object, based on the first time and the second time.

7. The ultrasonic measurement method according to claim 5 or 6, comprising:
after confirming that the distal end of the inner shaft and/or the distal end of the outer shaft contacts the surface of the object in the third step, a sixth step of inserting an insertion body into the object via the lumen of the inner shaft; and
after the sixth step, a seventh step of receiving, by the ultrasonic element, a third reflected wave reflected by the insertion body and measuring a third time from the transmission of the ultrasonic wave to a reception of the third reflected wave.

8. The ultrasonic measurement method according to claim 7, comprising:
after the seventh step, an eighth step of determining whether an insertion depth of the insertion body into the object is appropriate, based on the first time, the second time, and the third time.

9. A medical instrument, comprising:
the catheter according to any one of claims 1 to 3;
a therapeutic device being inserted into the lumen of the inner shaft and including a distal end portion capable of advancing further to a distal end side than the distal end of the inner shaft;
a first drive device that advances and retreats the catheter into and from a body cavity;
a second drive device that advances and retreats the therapeutic device; and
a control device that controls the first drive device and the second drive device.
